# EUROPEAN PATENT SPECIFICATION

(11) **EP 1 927 612 B1**
(45) Date of publication and mention of the grant of the patent: **07.10.2015**
(21) Application number: 07022352.4
(22) Date of filing: 17.11.2007
(51) Int. Cl.: C08G 65/26, C07C 41/03, C07C 43/11

(54) **Continuous process for the production of ethoxylates**
Kontinuierliches Verfahren zur Herstellung von Ethoxylaten
Procédés continus pour la production d'éthoxylates

(30) Priority: 01.12.2006 US 607349
(43) Date of publication of application: 04.06.2008
(60) Divisional application: 10003272.1
(73) Proprietor: Bayer MaterialScience LLC, Pittsburgh, PA 15205 (US)
(72) Inventor: McDaniel, Kenneth G., Charleston, WV 25311 (US); Reese, Jack R. II, Hurricane, WV 25526 (US)
(74) Representative: BIP Patents

(56) References cited:
- EP-A- 1 469 027
- WO-A-2005/103116
- DE-A1-102004 031 836
- JP-A- 6 016 806
- US-A- 5 689 012
- US-A- 5 919 988
- US-A1- 2002 198 413

## Description

### FIELD OF THE INVENTION

The present invention relates in general to polymerization, and more specifically, to improved processes for the production of ethoxylates useful in or as surfactants and detergents.

### BACKGROUND OF THE INVENTION

Alkyl and alkylaryl ethoxylates are widely used in the detergents industry with world consumption estimated to be in the range of 2,600 kilotons, including the alcohol ether sulfates, (based upon data contained in "Alternatives to Nonylphenol Ethoxylates, Review of Toxicity, biodegradation & Technical-Economic Aspects", Environment Canada, Final Report May 28, 2002 prepared by P.M. Campbell) found at http://www.c2p2online.com/documents/FinaINPEAIternativesPublicReport.pdf and the rate of growth between 2003 and 2008 is projected at about 3.4 percent.

Worldwide, the majority of ethoxylates used in detergents are produced via semi-batch processes utilizing base catalysis, typically potassium hydroxide ("KOH"). As such ethoxylates are commodity materials, the production economics and capability to manufacture the precursors are important determinants of profitability. Currently, only a small fraction of such surfactants are produced with specialized catalysts which give a narrow molecular weight distribution. Processes employing such "peaked catalysts" are reported to have higher manufacturing costs, likely related to higher catalyst costs and the need for catalyst removal following the ethoxylation process. Although the narrow distribution products from such processes can give higher performance in most applications, the cost driven focus of the detergent industry dictates the use of the KOH-catalyzed, wide distribution products because the benefits of enhanced performance are not sufficient to offset the increased costs.

The semi-batch process is the industry standard and although it is has been optimized and refined, there remain disadvantages to the use of this process. The semi-batch process operates with a headspace of ethylene oxide and an inerting gas and there are several nonproductive steps in the reactor sequence. Because pure ethylene oxide can present a hazard, the headspace must be inerted with nitrogen or low oxygen content gas and at the end of the cycle this inerting gas which may contain traces of ethylene oxide must be wasted. In the sequence of nonproductive steps: the alcohol is charged followed by catalyst and then the base is converted to the potassium or sodium alkoxide by stripping to remove water after the mixture is heated to process temperature. After oxide addition and digestion, the product is pumped from the reactor. Overall, the nonproductive steps can account for 50% of the reactor cycle time.

The use of double metal cyanide ("DMC") catalysts for the production of alkoxylates has been known since General Tire's development in the 1960's. In the 1970's, Herold in U.S. Pat. No. 3,829,505, described the preparation of high molecular weight diols, triols etc., using double metal cyanide catalysts. However, the catalyst activity, coupled with catalyst cost and the difficulty of removing catalyst residues from the polyol product, prevented commercialization of the products. There was limited utilization of the DMC technology until the 1990's when Le-Khac, in U.S. Pat. Nos. 5,470,813 and 5,482,908, demonstrated both improved catalysts and processes technologies that lowered the cost of production for polyols to be competitive with that for potassium hydroxide-based process for a wide range of polyols. However, even with these advancements, DMC catalyst technology has been applied mostly to the production of mixed oxide and all propylene oxide-based polyols.

Because of its unique features, the DMC catalyst provides a poor distribution of ethylene oxide when the equivalent weight of the base polyol is greater than about 800. The production of ethylene oxide ("EO") capped polyols suitable for use in polyurethanes requires either a two-stage system of DMC and potassium hydroxide or potassium hydroxide alone as the catalyst. Thus, although DMC catalysts are very effective for the production of ethoxylates when the starter equivalent weight is less than about 800, these capped low molecular weight polyols are not widely used for the production of polyurethanes.

The use of DMC as a catalyst for the production of semi-batch ethoxylates is disclosed in a number of patents and patent applications. For example, U.S. Pat. No. 6,821,308, issued Combs et al., teaches the alkoxylation of alcohols with DMC. Although they demonstrate the use of propylene oxide as the alkylene oxide, Combs et al. do not teach or suggest the use of pure ethylene oxide.

WO 00/14045 in the name of Grosch et al., teaches the preparation of ethoxylates of fatty alcohols using supported DMC catalysts along with propoxylation. Grosch et al. limit the range of alcohols used to C₆-C₂₄, thus eliminating the C₁-Cₛ alcohols likely because the lower molecular weight alcohols would act as inhibitors for catalyst activation in the semi-batch process.

Ruland et al., in Example 1 of U.S. Published Patent Application No. 2005/0215452, ethoxylate a 2-propylheptanol with five moles of ethylene oxide in the presence of a DMC catalyst. The polyethers of Ruland et al., have an ethylene oxide block followed by a mixed oxide block.

U.S. Published Patent Application No. 2005/0014979, in the name of Eleveld et al., discloses the use of DMC to prepare ethoxylated alcohols. It is likely that the process disclosed by Eleveld et al. will be unable to provide the economics needed to supply the majority of commodity surfactants, because many of the current highly optimized KOH processes operate in a heat exchange-limited mode and the use of another catalyst will not offset this limitation and this process retains the nonproductive steps of the semibatch process. Eleveld et al. report that DMC catalyzed semi-batch processes give narrow polydispersities in comparison with potassium hydroxide catalyzed processes; however, they do not disclose a method for surfactants having equivalent polydispersities.

Walker et al., in WO O1/04178, give several examples of ethoxylation. The batch or semibatch process was used for starters having a specific type of unsaturation which is modified if potassium hydroxide catalysis is used.

U.S. Pat. No. 6,642,423, issued to Clement et al., teaches ethoxylation with a DMC catalyst. The '423 patent discloses feeding a first block of ethylene oxide followed by other blocks of propylene oxide or mixed oxides. An activation period from several minutes to hours is required from this process.

Wulff et al., in U.S. Published Patent Application No. 2006/0052648, disclose various ethoxylates and other alkoxylates of 2-propylheptanol including certain process conditions for activation and mixture of inert gases with ethylene oxide. This application teaches the use of several nonproductive process steps.

WO 2006/002807 in the name of Ostrowski et al., discloses a continuous two-stage process for the production of polyols with mixed oxide segments. These products are developed for the slab polyurethane industry in which different polyether blocks within the same molecule may affect foam bun processing.

Wehmeyer et al., in U.S. Published Patent Application No.2006/0058482, disclose a continuous process for the production of mixed-oxide block polyols with a supported catalyst.

Thus, there remains a need for improved surfactant production processes. Such a process would be without starter molecular weight limitations and would not require a catalyst activation step to increase reactor productivity.

### SUMMARY OF THE INVENTION

Accordingly, the present invention provides a continuous process for the production of ethoxylates with a molecular weight distribution essentially equivalent to that of ethoxylates produced via a semi-batch process using basic catalysis (potassium hydroxide). The inventive continuous process produces an ethoxylate from a non-phenolic alcohol, which is a mixture of C₁₂ - C₁₅ monofunctional primary alcohols, in the presence of a double metal cyanide ("DMC") catalyst. The inventive multi-stage continuous process produces an ethoxylate product with a molecular weight distribution that is the same or narrower as that of the equivalent base-catalyzed ethoxylate. The essentially equivalent molecular weight distribution possible with the inventive multi-stage continuous process may provide advantages where the ethoxylate product is used in or as a surfactant.

These and other advantages and benefits of the present invention will be apparent from the Detailed Description of the Invention herein below.

### BRIEF DESCRIPTION OF THE FIGURES

The present invention will now be described for purposes of illustration and not limitation in conjunction with the figures, wherein:
Figure 1 is a gel permeation chromatograph comparing the polydispersity of two mixed oxide triols;
Figure 2 is a gel permeation chromatograph comparing the polydispersity of two all propylene oxide diols;
Figure 3a is a diagram of a continuous stirred reactor ("CSTR") partitioned with a perforated plate;
Figure 3b is a top view of a perforated plate;
Figure 4a is a diagram of a continuous stirred reactor ("CSTR") partitioned with a rotation disk;
Figure 4b is a top view of a rotation disk;
Figure 5 is a gel permeation chromatograph comparing the polydispersity of polyols produced by base catalysis, a DMC-catalyzed semi-batch process, a single stage DMC-catalyzed process and the inventive multi-stage DMC-catalyzed process; and
Figure 6 is a gel permeation chromatograph comparing the polydispersity of polyol produced base catalysis and a single stage DMC-catalyzed process.

### DETAILED DESCRIPTION OF THE INVENTION

Equivalent weights and molecular weights given herein in Daltons (Da) are number average equivalent weights and number average molecular weights respectively, unless indicated otherwise.

The present invention provides a multi-stage continuous process for the production of an ethoxylate involving preparing a mixture of a non-phenolic alcohol, which is a mixture of C₁₂ - C₁₅ monofunctional primary alcohols, and a double metal cyanide ("DMC") catalyst, establishing ethoxylation conditions in a first continuous stirred tank reactor ("CSTR"), continuously feeding ethylene oxide and the mixture of non-phenolic alcohol and DMC catalyst to the first CSTR reactor under conditions suitable to produce an ethoxylate, continuously feeding the reaction mixture from the first CSTR reactor and further ethylene oxide to a second CSTR reactor or to a tubular reactor under conditions suitable to produce an ethoxylate product and continuously withdrawing the ethoxylate product from the second CSTR reactor or tubular reactor to a collection vessel, wherein the ethoxylate product has a molecular weight distribution that is essentially equivalent to that of the same ethoxylate product produced by basic catalysis.

The present invention further provides an improved process for the production of a surfactant, the improvement involving including the ethoxylate product produced by the inventive multi-stage continuous process.

The present inventors have found that the polydispersities of the ethoxylates prepared with a continuous process using DMC catalysis are similar to those obtained using a semi-batch potassium hydroxide-catalyzed process. It is surprising that the inventive multi-stage continuous process is able to produce an ethoxylate with the same or narrower molecular weight distribution as compared to the equivalent commercial base-catalyzed ethoxylate.

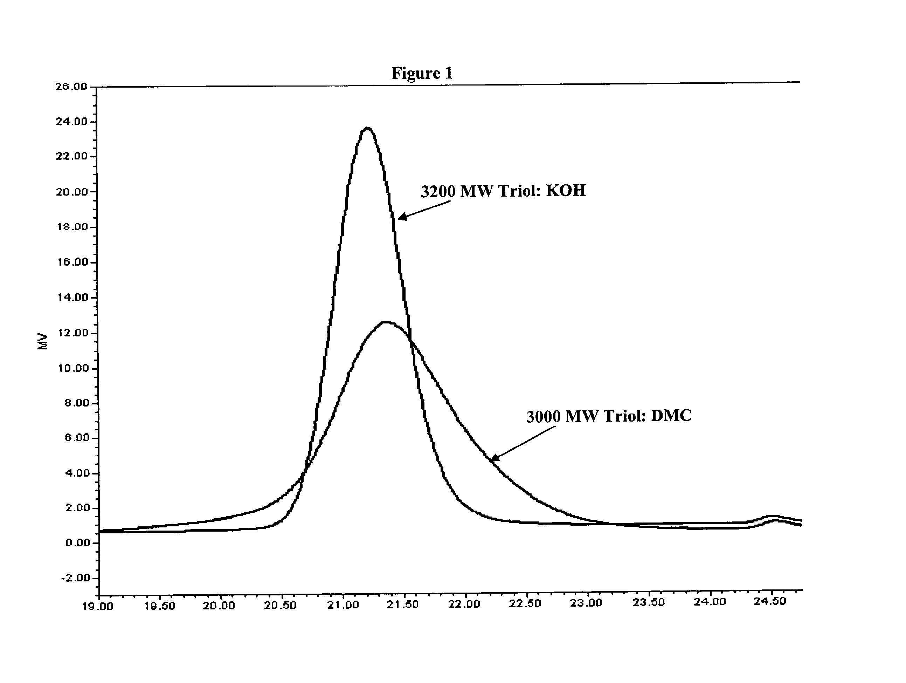

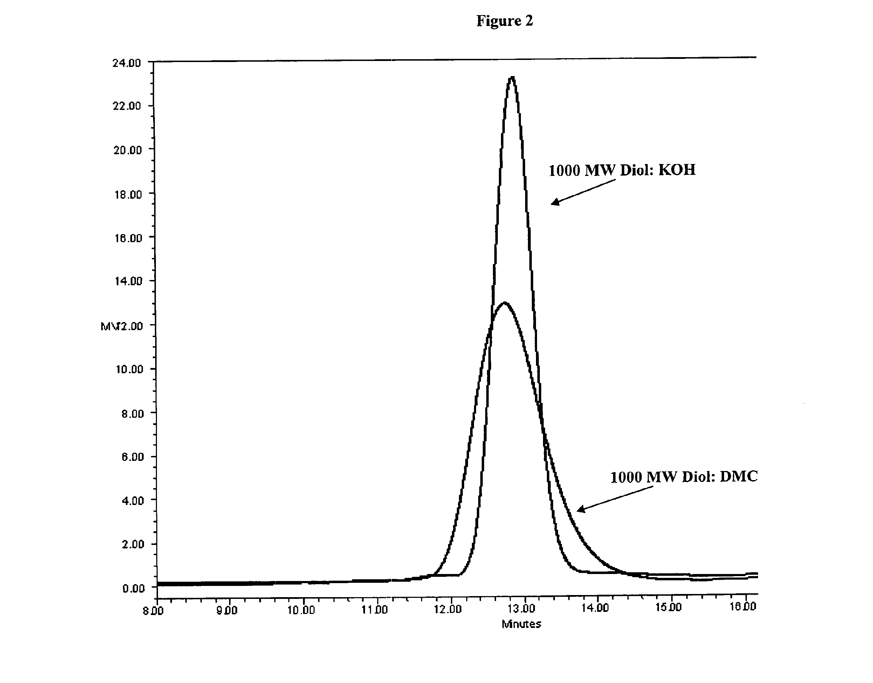

Such similar polydispersities are unexpected based on the published art and on the knowledge of those skilled in the art, regarding polydispersities of the polypropylene oxide polyols and mixed polyethylene oxide-polypropylene oxide polyols. With such polymers, the polydispersities are typically wider for the continuous products (DMC) process than the polydispersities of the products produced in the semibatch (KOH) process. Figure 1 compares the polydispersity of a 3,200 MW KOH-catalyzed triol with an 11.5 percent ethylene oxide content and a 3,000 MW DMC-catalyzed triol with an ethylene oxide content of 7.4 percent. Figure 2 compares the polydispersity of 1,000 MW all propylene oxide diols made by KOH catalysis and by DMC catalysis. In both cases, the DMC-catalyzed materials show a broader polydispersity.

As those skilled in the art are aware in transitioning from one manufacturing process to another, one of the key requirements is that the products have similar properties. It is known in the art that the relative surfactancy of a given weight of ethoxylates is related to the product distribution of the ethoxylates and thus it is desirable to maintain consistent properties. The inventive continuous process provides the desired product quality combined with increases in productivity.

The inventive continuous process eliminates the nonproductive sequences required for the semi-batch process. Once the startup is achieved, the reactor is fully utilized for alkoxylation. The catalyst, starter and ethylene oxide are continuously charged with no heat-up or water removal stages. The addition of the starter at ambient temperature is an advantage as the heat of reaction is used to bring it to process temperature. The catalyst in the reactor has on-going activity and new catalyst is continually activated as the process proceeds. The products flows from the reactor and the last traces of ethylene oxide are eliminated either in the piping system, in a short pipe reactor or in the product analysis tank. If the system is equipped with analytical instruments such as a near-infrared detector, product variability is low as incremental starter and ethylene oxide weight changes can be made to maintain product quality.

At the end of this process, the product may contain a low level of unreacted ethylene oxide. The oxide level continues to decrease as the product flows from the reactor by pipes to product analysis tanks. An alternative to the use of these lines would be a pipe or plug-flow reactor in which no oxide is added. In addition, any residual oxide would continue to be reduced while the ethoxylate was in the product tank.

Initiators or starters (the terms are used interchangeably herein) in the inventive multi-stage process are non-phenolic alcohols, wherein the non-phenolic alcohol is a mixture of C₁₂ - C₁₅ monofunctional primary alcohols.

The process of the present invention may employ any double metal cyanide ("DMC") catalyst. Double metal cyanide complex catalysts are non-stoichiometric complexes of a low molecular weight organic complexing agent and optionally other complexing agents with a double metal cyanide salt, e.g. zinc hexacyanocobaltate. Suitable DMC catalysts are known to those skilled in the art. Exemplary DMC catalysts include those suitable for preparation of low unsaturation polyoxyalkylene polyether polyols, such as disclosed in U.S. Pat. Nos. 3,427,256; 3,427,334; 3,427,335; 3,829,505; 4,472,560; 4,477,589; and 5,158,922. The DMC catalysts more preferred in the process of the present invention are those capable of preparing "ultra-low" unsaturation polyether polyols. Such catalysts are disclosed in U.S. Pat. Nos. 5,470,813 and 5,482,908, 5,545,601, 6,689,710 and 6,764,978. Particularly preferred in the inventive process are those zinc hexacyanocobaltate catalysts prepared by the processes described in U.S. Pat. No. 5,482,908.

The DMC catalyst concentration is chosen so as to ensure good control of the ethoxylation reaction under given reaction conditions. The catalyst concentration is preferably from 5 ppm to 1,000 ppm, more preferably in the range of from 10 ppm to 500 ppm, and most preferably in the range from 20 ppm to 100 ppm, based on the final ethoxylate weight. The ethoxylation in the process of the present invention may occur in the presence of DMC catalyst in an amount ranging between any combination of these values, inclusive of the recited values.

Although the inventors herein believe that the term "establishing ethoxylation conditions" in an oxyalkylation reactor is self-explanatory, such conditions are established when the reactor temperature, ethylene oxide pressure, catalyst level, degree of catalyst activation, presence of oxyalkylatable compounds within the reactor, etc., are such that upon addition of unreacted ethylene oxide to the reactor, ethoxylation takes place. By the term "continuously introducing" with respect to addition of ethoxylation oxide and starter herein is meant truly continuous, or an incremental addition which provides substantially the same results as continuous addition of these components. The terms "starter" and "initiator" as used herein are the same unless otherwise indicated.

The ethoxylates produced by the inventive process preferably have a number average molecular weight of from 200 Da to 20,000 Da, more preferably from 250 Da to 12,000 Da, most preferably from 350 Da to 650 Da. The ethoxylates produced by the inventive process may have a number average molecular weight ranging between any combination of these values, inclusive of the recited values. The ethoxylates produced by the inventive processes may preferably find use in or as surfactants.

The inventive multi-stage process takes place in a continuous stirred tank reactor (1^{st} stage) connected to a tubular (pipe or flow) reactor (2^{nd} stage) or connected to a second CSTR or other type of reactor.

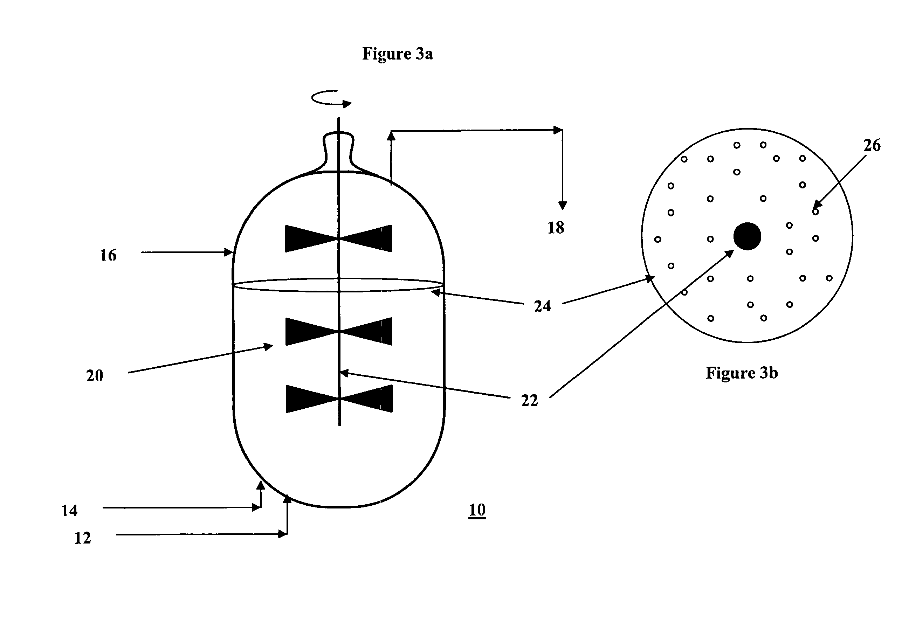

Figure 3a is a diagram of a partitioned CSTR reactor **10.** The CSTR reactor is physically partitioned into two (or more) compartments by a perforated plate **24** (or plates) shown in Fig. 3b. Catalyst/initiator slurry **12** and ethylene oxide **14** are fed into the lower compartment and reacted in that chamber to form the backbone of the polyol molecule. This polyol intermediate is forced to flow upward through the openings **26** of the perforated plate **24** to the upper compartment continuously. A separate feed **16** can be introduced into the upper compartment for a second reaction. The final product **18** is overflowed from the top of the reactor **10.** Additional stages may be added similarly, if required. The agitator blades **20** for both compartments are anchored in a common shaft **22.** Not shown are re-circulation loops (for cooling) and/or heat exchange surfaces (cooling jacket or coils) which may be added as needed.

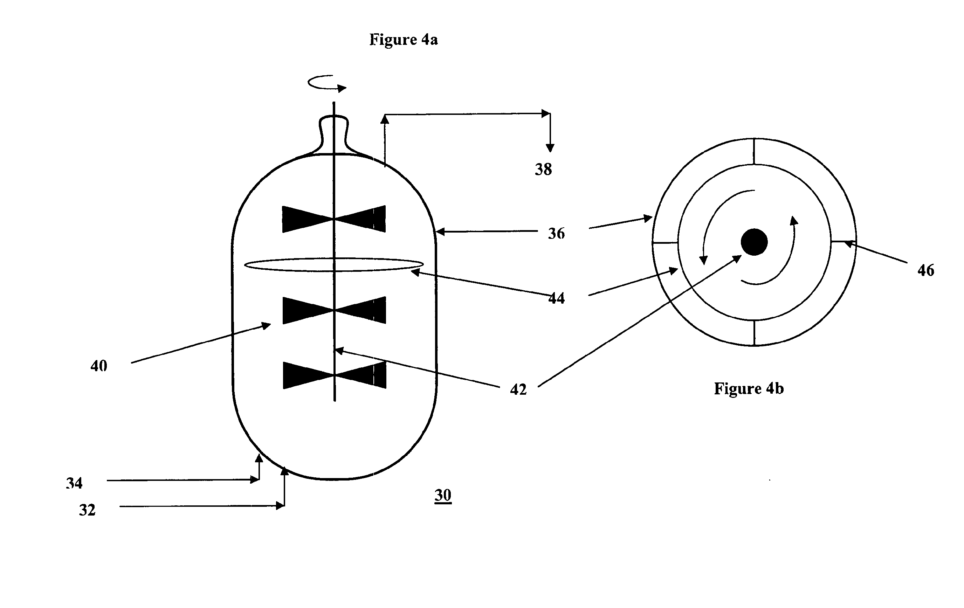

Alternatively, as shown in Figure 4a, a rotation disk **44** (or disks) may be anchored on the agitator shaft **42** and serve as a partition (or partitions) for the reactor **30.** As described above, catalyst/initiator slurry **32** and ethylene oxide **34** are fed into the lower compartment and reacted in that chamber to form the backbone of the polyol molecule. As shown in Figure 4b, the gap between the reactor wall **36** and the rim of disk **44** serves as an open space for liquid flow from the lower to the upper compartment. This design is more flexible because the location of the disk **44** (i.e. the volume ratio of the partitioned compartments) can be adjusted. A separate feed can be introduced into the upper compartment for a second reaction. The final product **38** is overflowed from the top of the reactor **30.** Baffles **46** may optionally be added.

It is expected that one skilled in the art could combine different types of reactors to produce a multistage reactor train which would give the desired product distribution. For example, other type of reactors that were originally developed for the semibatch alkoxylation processes could be easily modified for the continuous process including Venturi Loop reactors and spray tower loop reactors as given in: http://adt.lib.swin.edu.au/uploads/approved/adt-VSWT20050610.140607/public/ 02chapterl-4.pdf. Staged reactors and the compartmentalized reactors, such as those disclosed in U.S. Pat. No. 7,012,164, (*See* Figures 1-4) and variants thereof should be especially suitable for use in the inventive processes for producing ethoxylates.

### EXAMPLES

All quantities given in "parts" and "percents" are understood to be by weight, unless otherwise indicated. In all examples herein using a DMC catalyst, the catalyst was made according to U.S. Pat. No. 5,482,908. NEODOL 25, a blend of C₁₂, C₁₃, C₁₄ and C₁₅ high purity primary alcohols which is commercially available from Shell was used in the Examples.

### Examples C1, C2, 3, 4 and 5

Example C1 was a product from a commercial process utilizing KOH/NaOH as catalyst. Example C2 was a product from a DMC-catalyzed semi-batch process with a NEODOL 25 starter.

Example 3 (not according to the invention) was a product from a single stage DMC-catalyzed process with NEODOL 25 starter. A 35-hydroxyl number propoxylate of NEODOL 25 containing 30 ppm of DMC catalyst was charged to a one-gallon CSTR stainless steel reactor equipped with a mechanical agitator and slowly heated. Once the reactor temperature reached 130°C, an initial charge of ethylene oxide was charged to the reactor over several minutes. After 10 minutes, the pressure in the reactor decreased indicating that the DMC catalyst was active. The ethylene oxide feed was restarted and set at a rate of 19.4 g/min (equivalent to a two-hour residence time). After establishing the oxide feed, a feed containing NEODOL 25 and 131 ppm DMC catalyst was started at a rate of 10.2 g/min.

The DMC catalyst was added to the NEODOL 25 as a dry powder and remained dispersed in the NEODOL 25 by constant agitation of the NEODOL 25/ DMC catalyst feed vessel. The DMC concentration in the NEODOL 25 was sufficient to provide 45 ppm in the final product. When the pressure in the reactor reached 50 psia, a valve at the top of the reactor was opened to a back pressure regulator and the contents of the liquid full reactor were allowed to flow out of the reactor. The polyether coming out of the reactor was passed through a steam-heated line before being collected in a heated and stirred jacketed vessel. The ethylene oxide and NEODOL 25 /catalyst feeds continued for approximately 22 hours at which point both the feeds were stopped. A sample of the collected product had a measured hydroxyl number of 94.5 mg KOH/g and a polydispersity of 1.17.

In Example 4, a 114-hydroxyl number ethoxylate of NEODOL 25 containing 45 ppm of DMC catalyst was charged to a one-gallon CSTR stainless steel reactor equipped with a mechanical agitator and to a two-gallon CSTR stainless steel reactor equipped with a mechanical agitator and both reactors were slowly heated. The ethylene oxide feed to the one-gallon reactor was started and set at a rate of 16.7 g/min (equivalent to a 1.5 hr residence time in the one gallon reactor). After establishing the oxide feed, a feed containing NEODOL 25 and 144 ppm DMC catalyst was started at a rate of 23.95 g/min.

When the pressure in the reactor reached 50 psia, a valve at the top of the reactor was opened to a back pressure regulator and the contents of the liquid full reactor were allowed to flow out of the reactor. The polyether coming out of the one-gallon reactor was directed into the bottom of the two-gallon reactor. The ethylene oxide was then started to the two-gallon reactor at a rate of 16.7 g/min, equivalent to a 2.1 hr residence time in the two-gallon reactor. The polyether coming out of the liquid full two-gallon reactor was passed through a second back pressure regulator and onto a steam-heated line before being collected in a heated and stirred jacketed vessel. The ethylene oxide and NEODOL 25 /catalyst feeds continued for approximately 9 hours at which point the feeds to both reactors were stopped. A sample of the collected product had a hydroxyl number of 115 mgKOH/g and a polydispersity of 1.10.

For Example 5, the reaction from Example 4 was continued by re-charging the NEODOL 25 and DMC catalyst vessel with a mixture of NEODOL 25 and DMC catalyst that contained 176 ppm catalyst. The ethylene oxide (18.1 g/min) and NEODOL 25 /DMC catalyst mixture (18.9 g/min) feeds were restarted to the one-gallon reactor, equivalent to a residence time of 1.6 hours in the one-gallon reactor. A short time later, the ethylene oxide feed (17.9 g/min) to the two-gallon reactor was restarted, equivalent to a 2.2 hr residence time in the two-gallon reactor. The polyether was continuously removed from the two-gallon reactor and collected in a manner similar to Example 4. The feeds were continued for 9 hours at which point the feeds to both reactors were stopped. A sample of the collected product had a hydroxyl number of 99.4 mgKOH/g and a polydispersity of 1.09.

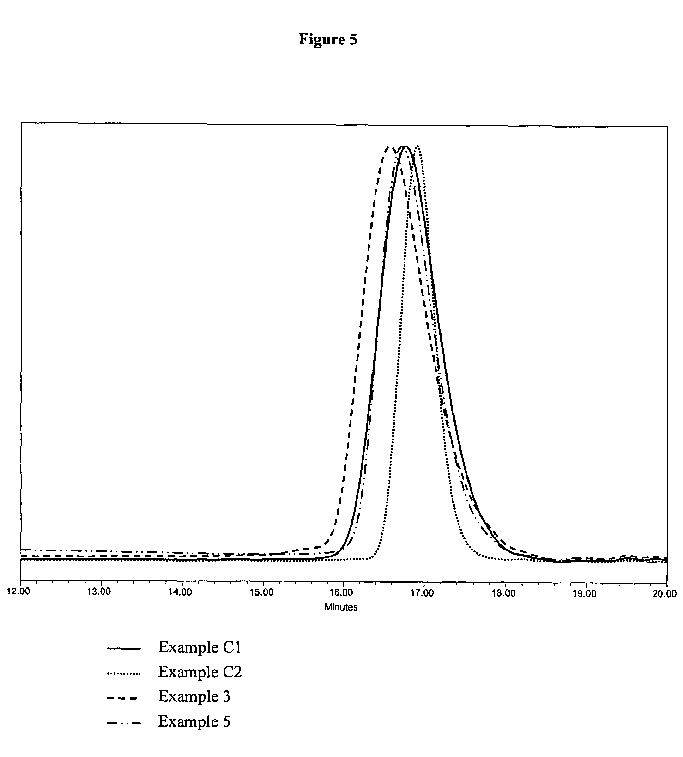

As will be apparent to one skilled in the art from a review of Table I below and Figure 5, the polydispersity of the polyols produced by the inventive multi-stage process (Example 5, dashed line with two dots) is essentially equivalent (i.e., no broader) than that of the base catalyzed control (Example C1, solid line). The polyol produced by the DMC-catalyzed single stage process (Example 3, dashed line) had a slightly broader polydispersity than that of the base catalyzed control (Example C1, solid line). Example C2 (dotted line) shows the polydispersity of a DMC-catalyzed semi-batch process produced polyol.

**Table I**

| | **Ex. C1** | **Ex. C2** | **Ex. 3** | **Ex. 4** | **Ex. 5** |
|---|---|---|---|---|---|
| moles EO/mole alcohol | 9 | 9 | 9 | 6.6 | 9 |
| process characterization | commercial | semi-batch | single stage | multi-stage continuous | multi-stage continuous |
| Continuous starter (Sc) | none | none | NEODOL25 | NEODOL25 | NEODOL25 |
| Catalyst Type | KOH/NaOH | DMC | DMC | DMC | DMC |
| Catalyst Conc. in Sc (ppm) | ----- | ----- | 87 | 144 | 176 |
| Final Catalyst (ppm) | ----- | 30 | 30 | 60 | 60 |
| Starter Feed rate (g/min) | ----- | ----- | 10.2 | 23.95 | 18.9 |
| Hydroxyl No. (mg KOH/g) | 93.4 | 90.1 | 94.5 | 115 | 99.4 |
| Viscosity @ 60°C (cst) | 26.8 | 25.6 | 23.1 | 16.5 | 21.5 |
| MW distribution Dispersity | 1.13 | 1.06 | 1.17 | 1.1 | 1.085 |
| Mn | 634 | 612 | 711 | 556 | 633 |
| Mw | 715 | 647 | 831 | 611 | 687 |
| Mp | 734 | 644 | 868 | 638 | 724 |
| Mz | 792 | 728 | 964 | 663 | 737 |

### Examples C6 and 7

Example C6 was the product from a commercial process utilizing KOH/NaOH as catalyst.

In Example 7 (not according to the invention), the reaction from Example 3 was continued by re-charging the NEODOL 25 and DMC catalyst vessel with a mixture of NEODOL 25 and DMC catalyst that contained 108 ppm catalyst. The ethylene oxide was re-started at a rate of 17.3 g/min and the NEODOL 25 /DMC catalyst mixture was fed at 12.4 g/min, equivalent to a two-hour residence time. The polyether was continuously removed from the reactor and collected in a manner similar to Example 3. The feeds were continued for 18 hours at which time the reaction was stopped. A sample of the collected product had a hydroxyl number of 114 mgKOH/g and a polydispersity of 1.14.

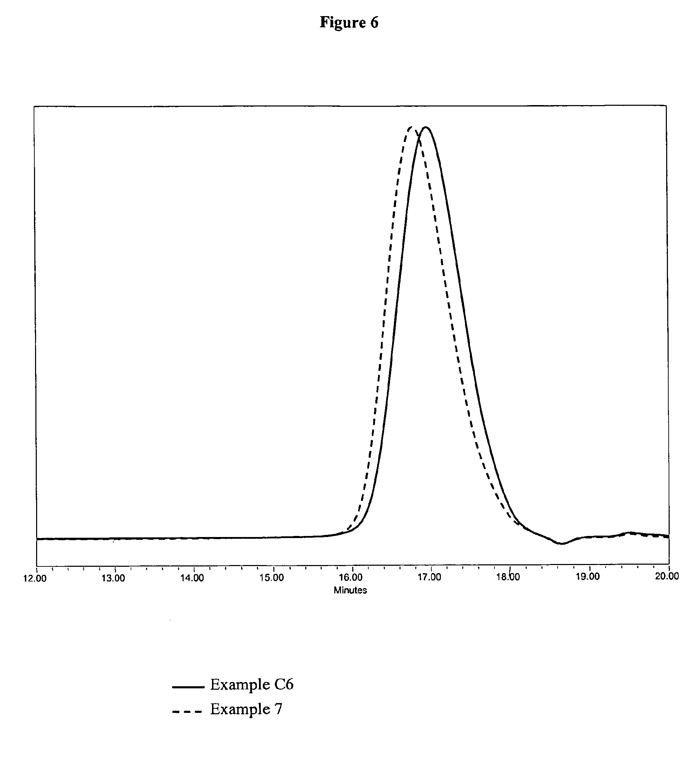

As can be appreciated by those skilled in the art upon review of Table II below and of Figure 6, the ethoxylate product produced by the single stage process (Example 7, dashed line) had a polydispersity that was about the same as that of the base catalyzed product (Example C6, solid line).

**Table II**

| | **Ex. C6** | **Ex. 7** |
|---|---|---|
| moles EO/mole alcohol | 6.6 | 6.6 |
| process characterization | commercial | single stage |
| Continuous starter (Sc) | none | NEODOL25 |
| Catalyst Type | KOH/NaOH | DMC |
| Catalyst Conc. in Sc (ppm) | **-**---- | 108 |
| Final Catalyst (ppm) | ----- | 45 |
| Starter Feed rate (g/min) | ----- | 12.4 |
| Hydroxyl No. (mg KOH/g) | 118 | 114 |
| Viscosity @ 60°C (cst) | 15.9 | 19 |
| MW distribution Dispersity | 1.13 | 1.14 |
| Mn | 538 | 601 |
| Mw | 610 | 687 |
| Mp | 614 | 718 |
| Mz | 687 | 774 |

The amount of unreacted alcohol remaining in an alkyl ethoxylate is an important parameter because these alcohols are reported to more odorous than the corresponding ethoxylates therefore, it is important to minimize any odor. The relative amounts of these residual alcohols are given below in Table III for some of the polyols produced according to the processes of the Examples. These data show the relative amounts of unreacted alcohols remaining in the products produced by the commercial (potassium hydroxide-catalyzed semi-batch process), the single-stage DMC-catalyzed continuous process and the inventive multi-stage DMC-catalyzed continuous process.

As can be appreciated by reference to Table III below, although the single stage DMC-catalyzed continuous process (Examples 3 and 7) produced somewhat higher levels of unreacted alcohol than were found in the commercial, base-catalyzed semi-batch, processes (Comparative Examples 1 and 6), the inventive multi-stage DMC-catalyzed continuous process produced amounts (Example 5) that were less than or equivalent to the amounts found in the commercial products (Comparative Examples 1 and 6).

Another potential advantage of the inventive multi-stage process is that the distributions had a slight "peaked shape" in which the amounts of the homologs around the targets of 6.5 and 9 moles of EO were higher than in the commercial products (*See*, Cox, M.F., "Ethylene Oxide Derived Surfactants", Proceeding of the 3rd World Conference on Detergents (1993)).

**Table III**

| | **Ex. C1** | **Ex. 3** | **Ex. 5** | **Ex. C6** | **Ex. 7** |
|---|---|---|---|---|---|
| Process/ Product | Commercial C₁₃-9 EO | Single-stage C₁₃-9 EO | multi-stage C₁₃-9 EO | Commercial C₁₃-6.6 EO | Single-stage C₁₃-6.6 EO |
| | **% Area** | **% Area** | **% Area** | **% Area** | **% Area** |
| EO Analog | | | | | |
| C₁₃ Alcohol | 1.0 | 2.7 | 1.0 | 2.4 | 4.4 |
| 1EO | N.D. | N.D. | N.D. | 5.7 | N.D. |
| 2EO | | | | | |
| 3EO | 5.3 | 7.3 | 4.2 | 9.2 | 8.4 |
| 4EO | 7.2 | 7.6 | 5.4 | 10.6 | 8.8 |
| 5EO | 8.1 | 7.8 | 6.4 | 10.9 | 9.3 |
| 6EO | 8.8 | 7.9 | 7.5 | 10.4 | 9.5 |
| 7EO | 9.5 | 7.8 | 7.9 | 9.9 | 9.5 |
| 8EO | 9.9 | 7.8 | 8.9 | 9.5 | 9.3 |
| 9EO | 9.5 | 7.9 | 9.8 | 8.3 | 9.2 |
| 10RO | 8.9 | 7.9 | 10.0 | 7.4 | 8.9 |
| 11RO | 8.2 | 7.5 | 9.9 | 5.8 | 7.7 |
| 12EO | 7.1 | 7.1 | 9.0 | 4.4 | 6.5 |
| 13EO | 5.8 | 6.3 | 7.6 | 3.1 | 5.0 |
| 14EO | 4.5 | 5.4 | 5.9 | 2.1 | 3.7 |
| 15EO | 3.4 | 4.5 | 4.0 | 1.4 | 2.6 |
| 16EO | 2.4 | 4.0 | 2.5 | 0.8 | 1.8 |
| 17EO | 1.5 | 3.3 | 1.1 | 0.5 | |

| | | | | | |
|---|---|---|---|---|---|
| N.D. - not determined | | | | | |

## Claims

1. A multi-stage continuous process for the production of an ethoxylate comprising:
preparing a mixture of a non-phenolic alcohol and a double metal cyanide ("DMC") catalyst;
establishing ethoxylation conditions in a first continuous stirred tank reactor ("CSTR");
continuously feeding ethylene oxide and the mixture of non-phenolic alcohol and DMC catalyst to the first CSTR reactor under conditions suitable to produce an ethoxylate;
continuously feeding the reaction mixture from the first CSTR reactor and further ethylene oxide to a second CSTR reactor or to a tubular reactor under conditions suitable to produce an ethoxylate product; and
continuously withdrawing the ethoxylate product from the second CSTR reactor or tubular reactor to a collection vessel,wherein the non-phenolic alcohol is a mixture of C₁₂ - C₁₅ monofunctional primary alcohols.

2. The multi-stage continuous process according to Claim 1, wherein the tubular reactor is a pipe reactor or a plug flow reactor.

3. The multi-stage continuous process according to Claim 1 further including a step of continuously feeding the product from the second CSTR reactor or tubular reactor to a third reactor under conditions suitable to produce an ethoxylate product.

4. The multi-stage continuous process according to Claim 3, wherein the third reactor is a CSTR reactor or a tubular reactor.

## Patentansprüche

1. Mehrstufiges kontinuierliches Verfahren zur Herstellung eines Ethoxylats, dass Folgendes umfasst:
Herstellung einer Mischung eines nichtphenolischen Alkohols und eines Doppelmetallcyanid-Katalysators ("DMC"-Katalysators);
Einstellen von Ethoxylierungsbedingungen in einem ersten kontinuierlich betriebenen Rührkessel (continuous stirred tank reactor, "CSTR");
kontinuierliches Zuführen von Ethylenoxid und der Mischung von nichtphenolischem Alkohol und DMC-Katalysator zum ersten CSTR-Reaktor unter Bedingungen, die zur Produktion eines Ethoxylats geeignet sind;
kontinuierliches Zuführen der Reaktionsmischung aus dem ersten CSTR-Reaktor und von weiterem Ethylenoxid zu einem zweiten CSTR-Reaktor oder einem Tubularreaktor unter Bedingungen, die zur Produktion eines Ethoxylat-Produkts geeignet sind; und
kontinuierliches Abziehen des Ethoxylat-Produkts aus dem zweiten CSTR-Reaktor bzw. Tubularreaktor in ein Sammelgefäß, wobei es sich bei dem nichtphenolischen Alkohol um ein Gemisch von monofunktionellen primären C₁₂-C₁₅-Alkoholen handelt.

2. Mehrstufiges kontinuierliches Verfahren nach Anspruch 1, wobei es sich bei dem Tubularreaktor um einen Rohrreaktor oder einen Pfropfenströmungsreaktor handelt.

3. Mehrstufiges kontinuierliches Verfahren nach Anspruch 1, ferner umfassend einen Schritt des kontinuierlichen Zuführens des Produkts aus dem zweiten CSTR-Reaktor bzw. Tubularreaktor zu einem dritten Reaktor unter Bedingungen, die zur Produktion eines Ethoxylat-Produkts geeignet sind.

4. Mehrstufiges kontinuierliches Verfahren nach Anspruch 3, bei dem es sich bei dem dritten Reaktor um einen CSTR-Reaktor oder Tubularreaktor handelt.

## Revendications

1. Procédé continu à étages multiples pour la production d'un éthoxylate comprenant :
la préparation d'un mélange d'un alcool non phénolique et d'un catalyseur de cyanure à double métal (« DMC ») ;
l'établissement de conditions d'éthoxylation dans un premier réacteur continu à cuve agitée (« CSTR ») ;
l'alimentation en continu d'oxyde d'éthylène et le mélange de l'alcool non phénolique et du catalyseur DMC dans le premier réacteur CSTR dans des conditions adaptées pour produire un éthoxylate ;
l'alimentation en continu du mélange de réaction provenant du premier réacteur CSTR et d'oxyde d'éthylène supplémentaire dans un deuxième réacteur CSTR ou dans un réacteur tubulaire dans des conditions adaptées pour former un produit éthoxylate ; et
le transfert en continu du produit éthoxylate depuis le deuxième réacteur CSTR où le réacteur tubulaire vers une cuve de collecte, dans lequel l'alcool non phénolique est un mélange d'alcools primaires monofonctionnels en C₁₂-C₁₅.

2. Procédé continu à étages multiples selon la revendication 1, dans lequel le réacteur tubulaire est un réacteur à tuyaux ou un réacteur à flux piston.

3. Procédé continu à étages multiples selon la revendication 1 comprenant en outre une étape d'alimentation en continu du produit provenant du deuxième réacteur CSTR ou du réacteur tubulaire dans un troisième réacteur dans des conditions adaptées pour former un produit éthoxylate.

4. Procédé continu à étages multiples selon la revendication 3, dans lequel le troisième réacteur est un réacteur CSTR ou un réacteur tubulaire.
